# EUROPEAN PATENT SPECIFICATION

(11) **EP 0 530 314 B1**
(45) Date of publication and mention of the grant of the patent: **02.11.1994**
(21) Application number: 91911530.3
(22) Date of filing: 03.05.1991
(51) Int. Cl.: C11D 1/75, C07C 291/04

(54) **AMINE OXIDE COMPOSITION AND PROCESS**
AMINOXIDZUSAMMENSETZUNG UND VERFAHREN ZUR HERSTELLUNG
COMPOSITION D'OXYDES D'AMINES ET LEUR PROCEDE DE PREPARATION

(30) Priority: 17.05.1990 US 524497; 17.05.1990 US 524569; 18.05.1990 US 525070; 21.05.1990 US 525984
(43) Date of publication of application: 10.03.1993
(73) Proprietor: ALBEMARLE CORPORATION, Richmond, Virginia 23219 (US)
(72) Inventor: BORLAND, James, E., Baton Rouge, LA 70815 (US); SAUER, Joe, D., Baton Rouge, LA 70816 (US); SMITH, Kim, R., Baton Rouge, LA 70810 (US)
(74) Representative: UEXKÜLL & STOLBERG
(86) International application number: US9103061
(87) International publication number: WO9118079

(56) References cited:
- EP-A- 0 028 038
- EP-A- 0 224 662
- US-A- 4 276 205

## Description

### Field of Invention

This invention relates to alkylamine oxide compositions and more particularly to such compositions suitable for incorporation into water-sensitive detergent formulations and to processes for preparing them.

### Background

As disclosed in Kirk-Othmer, Encyclopedia of Chemical Technology, Third Edition, Volume 2, pp. 259-271, it is known that amine oxides which are useful in various applications, such as detergent and shampoo formulations, can be prepared by reacting non-heterocyclic tert-amines with aqueous hydrogen peroxide in a solvent such as water, a lower alcohol, acetone, or acetic acid.

European Patent Application 0401503 (Smith et al.) teaches that it is also possible to prepare the amine oxides as solid, non-hygroscopic dihydrates by conducting their syntheses in organic solvents, such as esters, hydrocarbons, and highly polar solvents, in which the products are soluble at reaction temperatures but insoluble at a lower temperature.

These amine oxides, which are generally trialkylamine oxides, particularly trialkylamine oxides containing both short and long alkyl groups, are sometimes utilizable as prepared. However, the solid amine oxides present handling problems; and the solvents used in the known syntheses of amine oxides, especially the flammable solvents and water, are apt to be undesirable in some applications. Moreover, even when not undesirable because of making a harmful contribution, such as an excess of water in a water-sensitive formulation, the solvents have the unattractive feature of contributing weight and bulk to amine oxide-containing formulations without serving a useful purpose therein.

It would be advantageous to be able to provide alkylamine oxide compositions which could be poured or pumped from the vessels in which they are prepared to combine them with other ingredients of formulations in which they are to be incorporated, e.g., water-sensitive formulations, without having them in conjunction with solvents that would serve no useful function in the formulations and could be deleterious.

U. S. Patent 3,565,810 (Mausner et al.) shows a recognition of some of these problems and an attempt to solve them, but its process uses so much water that its products have too much unnecessary bulk and weight and would not be suitable for incorporation into water-sensitive formulations.

European Patent Application 0028038 (Leikhim et al.) teaches isotropic liquid detergent compositions comprising as five essential ingredients (1) an amine oxide surfactant, (2) an ethoxylated nonionic surfactant, (3) a water-soluble sequestering detergency builder, (4) a hydrophilic surface-active agent, and (5) water.

European Patent Application 0224662 (Legrand et al.) discloses the oxidation of tertiary aromatic amines with hydrogen peroxide in the presence of a selenium catalyst and a solvent, such as polyethylene glycol.

### Summary of Invention

It has now been found that compositions having a high surfactant content can be obtained by conducting at least the latter part of the reaction of a tert-amine with aqueous hydrogen peroxide to form an alkylamine oxide in the presence of a normally liquid polyethylene glycol or a nonionic surfactant as the sole organic solvent; at least a portion of any nonionic surfactant being a compound corresponding to one of the formulas Z[OC(Z')HCH₂]ₘOH, T[OC(Z')HCH₂]ₘOH, and Z(CO)NZZ'', in which T is an alkylphenyl group in which the alkyl group contains 4-30 carbons, Z is an alkyl group containing 4-30 carbons, Z' is hydrogen or an alkyl or hydroxyalkyl group containing 1-3 carbons, Z'' is a hydroxyalkyl group containing 1-3 carbons, and m is an integer of 1-100.

It has also been found that the use in the process of a fairly highly concentrated hydrogen peroxide solution and/or the removal of at least a portion of the water from the final reaction mixture can provide a pourable liquid blend of a tert-amine oxide and a solvent consisting essentially of a normally liquid polyethylene glycol or nonionic surfactant solvent and 0-10% by weight of water, a blend which is suitable for easy incorporation into water-sensitive formulations, such as bar soaps and detergent compositions.

### Detailed Description

The amine used in the practice of the invention may be any tert-amine that can be oxidized to a tert-amine oxide with aqueous hydrogen peroxide. Such amines are well known and include a variety of tert-amines. However, they are generally trialkylamines corresponding to the formula RR'R''N wherein R, R', and R'' are primary alkyl groups containing 1-30 carbons, preferably such trialkylamines in which R is methyl or ethyl, R' is an alkyl group containing 6-20 carbons, and R'' is independently selected from methyl, ethyl, and alkyl groups containing 6-20 carbons.

Exemplary of the tert-amines that may be used are trimethylamine, triethylamine, N-isobutyldimethylamine, trihexylamine, N,N-dimethyl-2-ethylhexylamine, N-eicosyldimethylamine, N-isobutyl-N-triacontylmethylamine, tri-2-hydroxyethylamine, N-dodecyldi-2-hydroxyethylamine, N,N-didecyl-2-hydroxyethylamine, and, more preferably, the N-alkyldimethyl-, N-alkyldiethyl-, N-alkyl-N-ethylmethyl-, N,N-dialkylmethyl-, and N,N-dialkylethylamines in which the alkyl groups are hexyl, octyl, decyl, dodecyl, tetradecyl, hexadecyl, octadecyl, and/or eicosyl, as well as mixtures of such amines.

The aqueous hydrogen peroxide which is reacted with the tert-amine may have a concentration of 1-99% by weight but, in a preferred embodiment of the invention, has a concentration of 50-70% by weight. The more dilute solutions are apt to contribute more water to the product than is desired in many instances, but the excess water may be removed from the product after completion of the reaction in those instances.

As in conventional tert-amine/hydrogen peroxide reactions, the amount of hydrogen peroxide employed should be at least the stoichiometric amount but generally not more than a 15% molar excess; and the reaction is conducted by adding the aqueous hydrogen peroxide to the amine, preferably at a controlled rate and preferably in the presence of carbon dioxide or a chelating agent, such as diethylenetriaminepentaacetic acid or ethylenediaminetetraacetic acid, at a temperature of 20-100° C, preferably 60-80° C; and the reaction temperature is maintained for 1-24 hours.

The process of the invention differs from the conventional processes in that at least the latter part of the reaction is conducted in the presence of a normally liquid polyethylene glycol or a nonionic surfactant as the sole organic solvent. Although the presence of such a solvent in the initial reaction mixture would not prevent the reaction from occurring, it is generally preferred to initiate the reaction in the absence of the organic solvent and then to add it gradually during the reaction so as to maintain the reaction mixture stirrable and/or so as to provide an amine oxide/nonionic surfactant weight ratio of 0.1-10/1 in the final reaction mixture.

When a polyethylene glycol solvent is employed in the process, it is one that is normally liquid, i.e., a polyethylene glycol or mixture of polyethylene glycols having average molecular weights such as to be liquid at ambient temperature.

When a nonionic surfactant solvent is employed in the process, it is a solvent that contains a compound corresponding to the one of the formulas Z-[OC(Z')HCH₂]ₘOH, T[OC(Z')HCH₂]ₘOH, and ZC(O)NZZ'', in which T is an alkylphenyl group in which the alkyl group contains 4-30 carbons, Z is an alkyl group containing 4-30 carbons, Z' is hydrogen or an alkyl or hydroxyalkyl group containing 1-3 carbons, Z'' is a hydroxyalkyl group containing 1-3 carbons, and m is an integer of 1-100. This compound may comprise only a portion of the nonionic surfactant solvent. However, in a preferred embodiment of the invention, more than 50% by weight of the nonionic surfactant solvent, preferably at least substantially all of it, is composed of this compound.

Nonionic surfactants corresponding to the first of the above formulas are well-known compounds which can be prepared by reacting ethylene oxide or an alkyl- or hydroxyalkyl-substituted ethylene oxide with an alkanol containing 4-30 carbons or with a mixture of such alkanols. For use in the present invention, these compounds should be liquids; and the preferred compounds are those in which m is an integer not higher than about 50, preferably 2-15. Also preferred are the compounds prepared from ethylene oxide, i.e., those in which Z' is hydrogen. Particularly good results have been obtained by the use of a mixture of ethoxylates of hexanol and decanol, e.g., such a mixture in which the ethylene oxide unit/alkanol unit mol ratio is an average of three; but good results are also obtained by the use of other compounds corresponding to the above formula, including those formed from one or more other straight- or branched-chain alkanols containing 4-30 carbons, such as butanol, isobutanol, hexanol, octanol, decanol, dodecanol, tetradecanol, pentadecanol, hexadecanol, octadecanol, eicosanol, docosanol, tetracosanol, or triacontanol.

Nonionic surfactants corresponding to the second of the above formulas are well-known compounds which can be prepared by reacting ethylene oxide or an alkyl- or hydroxyalkyl-substituted ethylene oxide with an alkylphenol in which the alkyl group contains 4-30 carbons or with a mixture of such alkylphenols. For use in the present invention, these compounds should be liquids; and the preferred compounds are those in which m is an integer not higher than about 50, preferably 2-15. Also preferred are the compounds prepared from ethylene oxide, i.e., those in which Z' is hydrogen. Particularly good results have been obtained by the use of a nonylphenol ethoxylate, e.g., such an ethoxylate in which the ethylene oxide unit/alkylphenol unit mol ratio is an average of nine; but good results are also obtained by the use of other compounds corresponding to the above formula, including those formed from one or more other alkylphenols in which the alkyl group contains 4-30 carbons, such as butyl-, isobutyl-, hexyl-, octyl-, nonyl-, decyl-, undecyl-, dodecyl-, tetradecyl-, hexadecyl-, octadecyl-, eicosyl-, docosyl-, tetracosyl-, and triacontylphenols.

Nonionic surfactants corresponding to the third of the above formulas are well-known compounds which can be prepared by reacting an amine corresponding to the formula Z'Z''NH, e.g., 2-hydroxyethylamine, di-2-hydroxyethylamine, or N-3-hydroxypropylmethylamine with a fatty acid corresponding to the formula ZCOOH, e.g., with ricinoleic, oleic, lauric, linoleic, coco, stearic, or capric acid. Particularly good results have been obtained with cocodiethanolamide.

When a surfactant corresponding to one of the above formulas is not the only component of the nonionic surfactant solvent, the remainder may be composed of one or more surfactants corresponding to one of the other formulas and/or one of more liquid nonionic surfactants of any other type. Exemplary of such other nonionic surfactants (which, like the aforementioned nonionic surfactants, are well known) are sorbitan oleates; sorbitan monolaurate; reaction products of sorbitan fatty acid esters with ethylene oxide; fatty acid (especially lauric) esters of glycols, such as ethylene glycol, diethylene glycol, and 1,2-propanediol; and compounds corresponding to the formula ZC(O)[OC(Z')HCH₂]ₘOH wherein Z, Z', and m have the meanings given above.

After completion of the reaction, the reaction mixture may be cooled to room temperature and, if necessary, then reheated to a fluidizing temperature when the reaction mixture is not fluid and it is desired to be able to pour or pump the reaction product from the vessel in which it was prepared. Alternatively, the reaction product may be poured or pumped from the reaction vessel before the temperature has been reduced to a point at which the product is no longer fluid when it is a product that is apt to gel at lower temperatures.

The alkylamine oxide solution formed by the process contains water because of the use of the aqueous hydrogen peroxide. Since a small amount of water can be tolerated even in water-sensitive formulations in which it might be desired to incorporate the amine oxide. It may not be necessary to remove any of the water in the solution. However, when the solution contains more water than is desired, it is removed from the reaction product by conventional means. The removal of the water may sometimes be accomplished by ordinary distillation, but the relatively low decomposition temperatures of some of the amine oxides makes it preferable to remove the water under reduced pressure to minimize the possibility of decomposing the product. Vacuum stripping is a particularly preferred method of removing at least a portion of the water from the product.

The invention is advantageous in that it provides alkylamine oxide compositions having a high surfactant content that makes them attractive for incorporation into detergents, not only because of their surfactant content but because of their being pourable or pumpable. Moreover, the compositions having low water contents have those advantages as well as an ability to be used in water-sensitive formulations, such as bar soaps and detergent concentrates; and the use of the polyethylene glycol serves to enhance the viscosity of personal care formulations instead of decreasing the viscosity as the use of an aqueous or alcoholic solvent would.

The following examples are given to illustrate the invention and are not intended as a limitation thereof.

### EXAMPLE I

A suitable reaction vessel was charged with 100g of N-tetradecyldimethylamine and 0.5g of diethylenetriaminepentaacetic acid. After the mixture was heated to 65 °C under a carbon dioxide atmosphere, 23g of 70% hydrogen peroxide (a 15% molar excess) was added over a period of five minutes while cooling to prevent the temperature from rising further. The temperature was then raised to 75 °C and held there for approximately seven hours while adding a polyethylene glycol having a molecular weight below 1000 as needed to facilitate stirring. By the end of the seven hours, a total of 55mL of the polyethylene glycol had been added, and the amine conversion was greater than 95%. The product was liquid at ambient temperature.

### EXAMPLE II

A suitable reaction vessel was charged with 100g of N-tetradecyldimethylamine and 0.5g of diethylenetriaminepentaacetic acid. After the mixture was heated to 65° C, 23g of 70% hydrogen peroxide (a 15% molar excess) was added over a period of five minutes while cooling to prevent the temperature from rising further. The temperature was then raised to 75° C and held there for approximately seven hours while adding a 3-mol ethoxylate of a hexanol/decanol blend as needed to facilitate stirring. By the end of the seven hours, a total of 55mL of the ethoxylate had been added, and the amine conversion was greater than 95%. The product was a water-white gel which melted to a pumpable fluid near 40° C.

### EXAMPLE III

A suitable reaction vessel was charged with 100g of N-tetradecyldimethylamine and 0.5g of diethylenetriaminepentaacetic acid. After the mixture was heated to 65° C, 23g of 70% hydrogen peroxide (a 15% molar excess) was added over a period of five minutes while cooling to prevent the temperature from rising further. The temperature was then raised to 75° C and held there for approximately 24 hours while adding a 9-mol ethoxylate of nonylphenol as needed during the first seven hours to facilitate stirring. By the end of the seven hours, a total of 45mL of the ethoxylate had been added, and the amine conversion was greater than 95%. The product was a gel which melted to a pumpable fluid near 40° C.

### EXAMPLE IV

A suitable reaction vessel was charged with 100g of N-tetradecyldimethylamine and 0.5g of diethylenetriaminepentaacetic acid. After the mixture was heated to 65°C, 23g of 70% hydrogen peroxide (a 15% molar excess) was added over a period of 15 minutes while cooling to prevent the temperature from rising further. The temperature was then raised to 75° C and held there for approximately seven hours while adding cocodiethanolamide as needed to facilitate stirring. By the end of the seven hours, a total of 50mL of the amide had been added, and the amine conversion was greater than 95%. The product was a liquid at ambient temperature.

## Claims

1. A pourable liquid blend consisting of:
(A) a tert-amine oxide corresponding to the formula RR'R''NO wherein R, R', and R'' are primary alkyl groups containing 1-30 carbons and
(B) a solvent consisting of:
(1) a normally liquid polyethylene glycol or
(2) a nonionic surfactant corresponding to one of the formulas Z[OC(Z')HCH₂]ₘOH, T[OC(Z')HCH₂]ₘOH, and Z(CO)NZ'Z'' in which T is an alkylphenyl group wherein the alkyl group contains 4-30 carbons, Z is an alkyl group containing 4-30 carbons, Z' is hydrogen or an alkyl or hydroxyalkyl group containing 1-3 carbons, Z'' is a hydroxyalkyl group containing 1-3 carbons, and m is an integer of 1-100, and
(3) 0-10% of water, based on the weight of the blend.

2. The blend of claim 1 wherein the amine oxide/polyethylene glycol or nonionic surfactant weight ratio is in the range of 0.1-10/1.

3. The blend of claim 1 or 2 wherein the tert-amine oxide is a compound corresponding to the formula RR'R''NO in which R is methyl or ethyl, R' is a primary alkyl group containing 6-20 carbons, and R'' is independently selected from methyl, ethyl, and primary alkyl groups containing 6-20 carbons.

4. The blend of any of the preceding claims wherein the solvent consists of a normally liquid polyethylene glycol and 0-10% of water, based on the weight of the blend.

5. The blend of any of claims 1-3 wherein the solvent consists of a nonionic surfactant and 0-10% of water, based on the weight of the blend.

6. The blend of claim 5 wherein the nonionic surfactant is at least one compound corresponding to the formula Z[OC(Z')HCH₂]ₘOH or to the formula T[OC(Z')HCH₂]ₘOH.

7. The blend of claim 5 wherein the nonionic surfactant is a compound corresponding to the formula Z(CO)NZ'Z''.

8. A process for preparing a tert-amine oxide by reacting a tert-amine corresponding to the formula RR'R''N wherein R, R, and R'' are primary alkyl groups containing 1-30 carbons with aqueous hydrogen peroxide, characterized in that at least the latter part of the reaction is conducted in the presence of a normally liquid polyethylene glycol or a nonionic surfactant as the sole organic solvent; at least a portion of any nonionic surfactant being a compound corresponding to one of the formulas Z[OC(Z')HCH₂]ₘOH, T[OC(Z')HCH₂]ₘOH, and Z(CO)NZ'Z'' in which T is an alkylphenyl group wherein the alkyl group contains 4-30 carbons, Z is an alkyl group containing 4-30 carbons, Z' is hydrogen or an alkyl or hydroxyalkyl group containing 1-3 carbons, Z'' is a hydroxyalkyl group containing 1-3 carbons, and m is an integer of 1-100.

9. The process of claim 8 wherein the tert-amine is reacted at a temperature of 60-80° C with at least a stoichiometric amount of an aqueous hydrogen peroxide solution having a concentration of 50-70% by weight in the initial absence of the polyethylene glycol or nonionic surfactant, which is added gradually during the reaction so as to maintain the reaction mixture stirrable.

## Patentansprüche

1. Vergießbare Flüssigkeitsmischung bestehend aus:
(A) einem tert-Aminoxid der Formel RR'R''NO, in der R, R' und R'' primäre Alkylgruppen mit 1 bis 30 Kohlenstoffatomen sind und
(B) einem Lösungsmittel aus:
(1) einem normalerweise flüssigen Polyethylenglykol oder
(2) einem nichtionischen oberflächenaktiven Mittel gemäß einer der Formeln Z[OC(Z')HCH₂]ₘOH, T[OC(Z')HCH₂]ₘ und Z(CO)NZ'Z'', in denen T eine Alkylphenylgruppe ist, in der die Alkylgruppe 4 bis 30 Kohlenstoffatome enthält, Z eine Alkylgruppe mit 4 bis 30 Kohlenstoffatomen, Z' Wasserstoff oder eine Alkyl- oder Hydroxyalklygruppe mit 1 bis 3 Kohlenstoffatomen, Z'' eine Hydroxyalkylgruppe mit 1 bis 3 Kohlenstoffatomen und m eine ganze Zahl von 1 bis 100 ist und
(3) 0 bis 10 % Wasser, bezogen auf das Gewicht der Mischung.

2. Mischung nach Anspruch 1, in der das Gewichtsverhältnis von Aminoxid zu Polyethylenglykol oder dem nichtionischen oberflächenaktiven Mittel im Bereich von 0,1 - 10 Zu l liegt.

3. Mischung nach Anspruch 1 oder 2, in der das tert-Aminoxid der Formel RR'R''NO entspricht, in der R Methyl oder Ethyl, R' eine primäre Alkylgruppe mit 6 bis 20 Kohlenstoffatomen und R'' unabhängig aus Methyl, Ethyl und primären Alkylgruppen mit 6 bis 20 Kohlenstoffatomen ausgewählt ist.

4. Mischung nach einem der vorstehenden Ansprüche, in der das Lösungsmittel aus einem normalerweise flüssigen Polyethylenglykol und 0 bis 10 % Wasser, bezogen auf das Gewicht der Mischung, besteht.

5. Mischung nach einem der Ansprüche 1 bis 3, in der das Lösungsmittel aus einem nichtionischen oberflächenaktiven Mittel und 0 bis 10 % Wasser, bezogen auf das Gewicht der Mischung, besteht.

6. Mischung nach Anspruch 5, in der das nichtionische oberflächenaktive Mittel mindestens eine Verbindung der Formel Z[OC(Z')HCH₂]ₘOH oder T[OC(Z')HCH₂]ₘ ist.

7. Mischung nach Anspruch 5, in der das nichtionische oberflächenaktive Mittel eine Verbindung der Formel Z(CO)NZ'Z'' ist.

8. Verfahren zur Herstellung eines tert-Aminoxids durch Umsetzung eines tert-Amins der Formel RR'R''N, in der R, R' und R'' primäre Alkylgruppen mit 1 bis 30 Kohlenstoffatomen sind, mit wäßrigem Wasserstoffperoxid, dadurch gekennzeichnet, daß zumindest der letztere Teil der Reaktion in Gegenwart eines normalerweise flüssigen Polyethylenglykols oder eines nichtionischen oberflächenaktiven Mittels als einziges organisches Lösungsmittels stattfindet, wobei mindestens ein Teil jedes nichtionischen oberflächenaktiven Mittels eine Verbindung einer der Formeln Z[OC(Z')HCH₂]ₘOH, T[OC(Z')HCH₂]ₘ und Z(CO)NZ'Z'' ist, worin T eine Alkylphenylgruppe ist, in der die Alkylgruppe 4 bis 30 Kohlenstoffatome enthält, Z eine Alkylgruppe mit 4 bis 30 Kohlenstoffatomen, Z' Wasserstoff oder eine Alkyl- oder Hydroxyalkylgruppe mit 1 bis 3 Kohlenstoffatomen, Z'' eine Hydroxyalkylgruppe mit 1 bis 3 Kohlenstoffatomen und m eine ganze Zahl von 1 bis 100 ist.

9. Verfahren nach Anspruch 8, bei dem das tert-Amin bei einer Temperatur von 60 bis 80°C mit mindestens einer stöchiometrischen Menge einer wäßrigen Wasserstoffperoxidlösung mit einer Konzentration von 50 bis 70 Gew.-% umgesetzt wird, wobei das Polyethylenglykol oder das nichtionische oberflächenaktive Mittel zu Anfang nicht vorhanden ist, sondern erst während der Reaktion allmählich zugesetzt wird, um das Reaktionsgemisch rührbar zu halten.

## Revendications

1. Mélange liquide apte à être versé, consistant en :
(A) un oxyde d'amine tertiaire répondant à la formule RR'R''NO dans laquelle R, R' et R'' représentent des groupes alkyle primaires contenant 1 à 30 atomes de carbone, et
(B) un solvant consistant en :
(1) un polyéthylèneglycol normalement liquide, ou
(2) un surfactant non ionique répondant à l'une des formules Z[OC(Z')HCH₂]ₘ, T[OC(Z')HCH₂]ₘOH et Z(CO)NZ'Z'', dans lesquelles T représente un groupe alkylphényle dans lequel le groupe alkyle contient 4 à 30 atomes de carbone, Z représente un groupe alkyle contenant 4 à 30 atomes de carbone, Z' représente l'hydrogène ou un groupe alkyle ou hydroxyalkyle contenant 1 à 3 atomes de carbone, Z'' représente un groupe hydroxyalkyle contenant 1 à 3 atomes de carbone, et m est un nombre entier de 1 à 100, et
(3) 0 à 10 % d'eau, sur la base du poids du mélange.

2. Mélange suivant la revendication 1, dans lequel le rapport pondéral oxyde d'amine/polyéthylèneglycol ou surfactant non ionique est compris dans l'intervalle de 0,1-10/1.

3. Mélange suivant la revendication 1 ou 2, dans lequel l'oxyde d'amine tertiaire est un composé répondant à la formule RR'R''NO dans laquelle R représente un groupe méthyle ou éthyle, R' représente un groupe alkyle primaire contenant 6 à 20 atomes de carbone, et R'' est choisi indépendamment entre des groupes méthyle, éthyle et alkyle primaire contenant 6 à 20 atomes de carbone.

4. Mélange suivant l'une quelconque des revendications précédentes, dans lequel le solvant consiste en un polyéthylèneglycol normalement liquide et 0 à 10 % d'eau, sur la base du poids du mélange.

5. Mélange suivant l'une quelconque des revendications 1 à 3, dans lequel le solvant consiste en un surfactant non ionique et 0 à 10 % d'eau, sur la base du poids du mélange.

6. Mélange suivant la revendication 5, dans lequel le surfactant non ionique consiste en au moins un composé répondant à la formule Z[OC(Z')HCH₂]ₘ ou à la formule T[OC(Z')HCH₂]ₘOH.

7. Mélange suivant la revendication 5, dans lequel le surfactant non ionique est un composé répondant à la formule Z(CO)NZ'Z''.

8. Procédé de préparation d'un oxyde d'amine tertiaire par réaction d'une amine tertiaire répondant à la formule RR'R''N dans laquelle R, R' et R'' représentent des groupes alkyle primaires contenant 1 à 30 atomes de carbone avec une solution aqueuse de peroxyde d'hydrogène, caractérisé en ce qu'au moins la dernière partie de la réaction est conduite en présence d'un polyéthylèneglycol normalement liquide ou d'un surfactant non ionique comme seul solvant organique ; au moins une partie de n'importe quel surfactant non ionique consistant en un composé répondant à une des formules Z[OC(Z')HCH₂]ₘ, T[OC(Z')HCH₂]ₘOH et Z(CO)NZ'Z'', dans lesquelles T représente un groupe alkylphényle dans lequel le groupe alkyle contient 4 à 30 atomes de carbone, Z représente un groupe alkyle contenant 4 à 30 atomes de carbone, Z' représente l'hydrogène ou un groupe alkyle ou hydroxyalkyle contenant 1 à 3 atomes de carbone, Z'' représente un groupe hydroxyalkyle contenant 1 à 3 atomes de carbone, et m est un nombre entier de 1 à 100.

9. Procédé suivant la revendication 8, dans lequel l'amine tertiaire est amenée à réagir à une température de 60 à 80°C avec au moins une quantité stoechiométrique d'une solution aqueuse de peroxyde d'hydrogène ayant une concentration de 50 à 70 % en poids en l'absence initiale du polyéthylèneglycol ou du surfactant non ionique, qui est ajouté progressivement au cours de la réaction de manière à maintenir le mélange réactionnel apte à l'agitation.
